# EUROPEAN PATENT APPLICATION

(11) **EP 4 249 090 A1**
(43) Date of publication of application: **27.09.2023**
(21) Application number: 20966984.5
(22) Date of filing: 25.12.2020
(51) Int. Cl.: A63B 69/00

(54) **EXERCISE MOTION ANALYSIS SYSTEM, EXERCISE MOTION ANALYSIS METHOD, AND EXERCISE MOTION ANALYSIS PROGRAM**

(71) Applicant: ASICS Corporation, Kobe-shi Hyogo 650-8555 (JP)
(72) Inventor: INOMATA, Takashi, Kobe-shi Hyogo 650-8555 (JP)
(74) Representative: Marks & Clerk LLP
(86) International application number: PCT/JP2020/048686
(87) International publication number: WO 2022/137498

(57) **Abstract**

In an exercise motion analysis system 100, an input unit 62 inputs motion data corresponding to multiple types of motion parameters indicating motion states of a user who is running. A selection unit 72 selects, from among multiple types of wearing tools, a type of a wearing tool commensurate with a runner type in which motion data of the user is classified, among multiple runner types classified in advance based on cluster analysis on motion data of multiple people. An output unit 74 outputs the type of a wearing tool selected as a wearing tool of which wearing is to be recommended to the user.

## Description

### TECHNICAL FIELD

The present invention relates to a technology for analyzing exercise motions.

### BACKGROUND ART

In recent years, with the increasing health consciousness of people and the improvement of various measurement technologies using information terminals and wearable devices, runners can easily obtain and record running information (time, distance, elevation, temperature, heart rate, and motion information including the cadence and stride) and can use the information for analysis of their performance (see Patent Literature 1, for example).

### PRIOR ART REFERENCE

### PATENT LITERTURE

Patent Literature 1: Japanese Unexamined Patent Application Publication No. 2018-126360

### SUMMARY OF INVENTION

### TECHNICAL PROBLEM

In order for runners to improve their performance (records) and reduce the risk of injuries (running injuries), it is desirable to select the most suitable shoes or products and training methods including strength training and practice menus for themselves. When ordinary runners purchase shoes and the like or decide training methods, they often make choices based on recommendations from acquaintances or information published in magazines, books, or on the Internet. However, it is difficult to objectively evaluate whether the selected products truly fit their running styles. Even if they feel no particular discomfort while actually wearing the products, ordinary runners cannot easily realize whether the products have led to enhancement of their performance. Especially in the current situation where many product models are on the market, there may be other product models more suitable for the runners.

The present invention has been made in view of such a problem, and a purpose thereof is to provide a technology for appropriately analyzing exercise motions and providing useful information to users.

### SOLUTION TO PROBLEM

To solve the problem above, an exercise motion analysis system according to one aspect of the present invention includes: an input unit that inputs motion data corresponding to multiple types of motion parameters indicating motion states of a user who is running; a selection unit that selects, from among multiple types of wearing tools, a type of a wearing tool commensurate with a runner type in which motion data of the user is classified, among multiple runner types classified in advance based on cluster analysis on motion data of multiple people; and an output unit that outputs the type of a wearing tool selected by the selection unit as a wearing tool of which wearing is to be recommended to the user.

The "wearing tools" may be running shoes to be worn during exercise, especially during running, and functional apparel including compression tights to be worn. The "motion parameters" may be parameters indicating running motions, such as the cadence, ground contact time, ground contact time rate, vertical stiffness, vertical motion, landing impact, kicking acceleration, amount of body drop, kicking phase duration, amount of braking, horizontal impact force, amount of pelvis rotation, braking time, and the like. The "index parameters" may be parameters indicating running performance, such as the heart rate, oxygen uptake, running time, running distance, speed, pace, presence or absence of pain, presence or absence of fatigue, and the like.

According to this aspect, with the motion data corresponding to multiple types of motion parameters indicating motion states of a user, the runner type of the user can be objectively understood, and shoes to be recommended can be selected more appropriately based on such a runner type objectively divided. Also, by classifying the runner types through cluster analysis, the type of a runner can be objectified with such a dividing method that is not artificial or uniform, and shoes can be selected more appropriately based on such a runner type objectively divided.

The exercise motion analysis system may further include a model storage unit that stores, for each of the multiple runner types, a prediction model created by machine learning using, as teacher data, motion data of multiple people classified in the runner type. Also, the exercise motion analysis system may further include an estimation unit that estimates the runner type by inputting motion data of the user into the prediction model.

The exercise motion analysis system may further include an analysis unit that performs cluster analysis on motion data of multiple people and classifies the motion data into multiple runner types, and a model creating unit that creates, for each of the multiple runner types, the prediction model by machine learning using, as teacher data, motion data of multiple people classified in the runner type.

The exercise motion analysis system may further include an item storage unit that stores a type of a wearing tool having a property of changing a motion parameter such as to conform to a tendency of multiple types of motion parameters of a runner type in motion data classified into the multiple runner types and thereby contributing to enhancement of exercise performance, as a type of a wearing tool commensurate with the runner type. The selection unit may select, from among the multiple types of wearing tools, a wearing tool commensurate with a runner type in which motion data of the user are classified, based on information stored in the item storage unit.

The input unit may further input index data corresponding to an index parameter that indicates exercise performance of the user in the running. The estimation unit may estimate, for each of the multiple classified runner types and based on a multiple regression analysis model for obtaining the degree of contribution of each motion parameter to enhancement of exercise performance with the multiple types of motion parameters in motion data of multiple people classified in a runner type set as the explanatory variables and with the index parameter in index data of multiple people classified in the runner type set as the objective variable, which motion parameter has a higher degree of contribution to enhancement of exercise performance for a user of the runner type. The selection unit may select a type of a wearing tool having a property of changing the estimated motion parameter and thereby contributing to enhancement of exercise performance, from among the multiple types of wearing tools.

Another aspect of the present invention also relates to an exercise motion analysis system. The exercise motion analysis system includes: an input unit that inputs motion data corresponding to multiple types of motion parameters indicating motion states of a user who is running; an analysis unit that performs cluster analysis on motion data of multiple people and classifies the motion data into multiple runner types; a model creating unit that creates, for each of the multiple runner types, the prediction model by machine learning using, as teacher data, motion data of multiple people classified in the runner type; an estimation unit that estimates the runner type by inputting motion data of the user into the prediction model; and an output unit that outputs information corresponding to a tendency of a value of the motion parameter in the estimated runner type. The "information corresponding to the type of the estimated motion parameter" means information that is useful to a user seeking to enhance his or her exercise performance and may include, for example, information that is helpful in selecting wearing tools such as shoes and also include information on training methods and competitions. The training methods may be running practice menus, such as speed training and hill training, in which the pace, distance, and course are specified, or may be reinforcement exercises other than running, such as strength training, stretching, and drills for acquiring movements.

Yet another aspect of the present invention relates to an exercise motion analysis method. The method includes: inputting, by a predetermined information acquiring means, motion data corresponding to multiple types of motion parameters indicating motion states of a user who is running; selecting, by predetermined processing performed by a computer and from among multiple types of wearing tools, a type of a wearing tool commensurate with a runner type in which motion data of the user is classified, among multiple runner types classified in advance based on cluster analysis on motion data of multiple people; and outputting, by a predetermined information outputting means, the type of the selected wearing tool as a wearing tool of which wearing is to be recommended to the user.

Optional combinations of the aforementioned constituting elements, and implementation of the present invention, including the constituting elements and expressions, in the form of methods, apparatuses, programs, transitory or non-transitory storage medium storing programs, or systems may also be practiced as additional modes of the present invention.

### ADVANTAGEOUS EFFECTS OF INVENTION

The present invention enables appropriate analysis of exercise motions to provide useful information to users.

### BRIEF DESCRIPTION OF DRAWINGS

FIG. 1 is a diagram that illustrates a basic configuration of an exercise motion analysis system.
FIG. 2 is a diagram that shows examples of motion data and index data.
FIG. 3 is a diagram that shows relationships between product models of shoes and the like and motion parameter changing properties of the respective product models.
FIG. 4 is a functional block diagram that shows basic configurations of a user terminal and an exercise motion analysis server.
FIG. 5 is a tree diagram that shows cluster analysis of motion data using the Ward's method.
FIG. 6 is a diagram that shows an example of a regression equation for each runner type.
FIG. 7 is a diagram that shows a first screen example for recommending shoes to a user.
FIG. 8 is a diagram that shows a second screen example for recommending shoes to a user.
FIG. 9 is a diagram that shows a third screen example for recommending shoes to a user.
FIG. 10 is a diagram that shows a fourth screen example for recommending shoes to a user.
FIG. 11 is a diagram that shows a screen example for suggesting improvement through stretching and training to a user.
FIG. 12 is a diagram that shows a screen example for suggesting participation in a marathon race to a user.
FIG. 13 is a diagram that shows a screen example for suggesting a practice menu to a user.
FIG. 14 is a flowchart that shows a process of selecting a wearing tool.

### DESCRIPTION OF EMBODIMENTS

In the present embodiment, based on various pieces of information acquired by a wearable device worn by a user during running, the runner type of the user is analyzed. Based on the runner type, shoes and functional apparel (hereinafter, "shoes" and "functional apparel" may also be collectively referred to as "shoes and the like" as appropriate) that can be expected to enhance the exercise performance are selected from multiple options and recommended to the user.

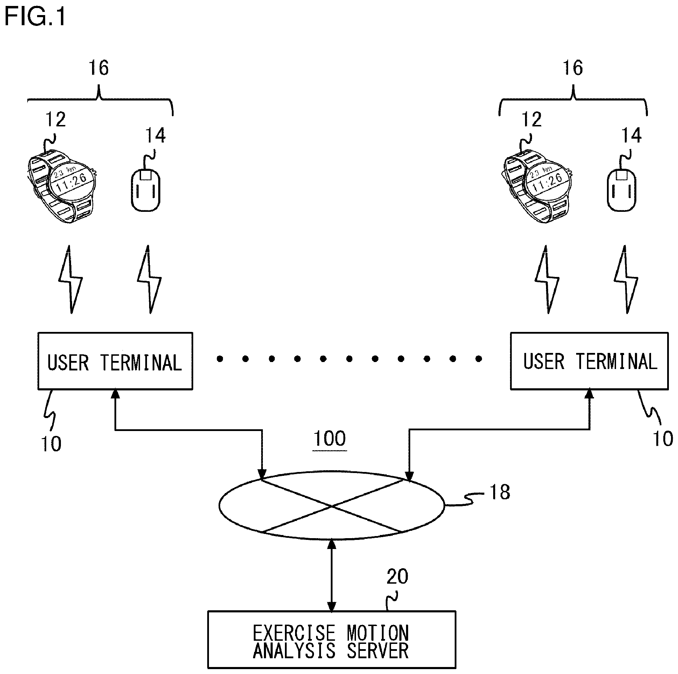

FIG. 1 illustrates a basic configuration of an exercise motion analysis system. A user performs running while wearing a wearable device 16, such as a running watch 12 and a motion sensor 14, on an arm or the waist and acquires various detection data with the running watch 12 and the motion sensor 14. The running watch 12 includes a positioning module and sensors, such as an acceleration sensor, a heart rate sensor, and a barometer, and acquires, as detection data, information including the date and time, position coordinates, altitude, heart rate, temperature, and cadence, for example. The motion sensor 14 acquires detection data detected by a 9-axis sensor, for example. As the wearable device 16, a motion sensor built into a mobile terminal such as a smartphone may also be used, or a chest strap type heart rate sensor or a motion sensor attached inside or outside a shoe may also be used.

The user synchronizes the detection data acquired by the wearable device 16 with a user terminal 10 via short-range wireless communication or the like. Based on the detection data acquired from the wearable device 16, the user terminal 10 acquires not only running log data, such as the running date and time, time, distance, altitude, speed, pace, and temperature, but also values of multiple types of motion parameters indicating motion states, such as the stride length, cadence, vertical motion ratio, ground contact time, ground contact time balance, landing impact, landing pattern, and pronation, and values of one or more types of index parameters indicating exercise performance, such as the heart rate and oxygen uptake. The user terminal 10 transmits the running log data, motion data corresponding to the motion parameters, and index data corresponding to the index parameters thus acquired to an exercise motion analysis server 20 via a network 18. The exercise motion analysis server 20 determines the runner type of the user based on the motion data received from multiple user terminals 10, selects shoes and the like of which use is to be recommended to the user based on the runner type, and recommends the shoes and the like to the user. Also, the exercise motion analysis server 20 provides, to the user, a suggestion based on the runner type.

Each user terminal 10 may be a personal computer or may be an information terminal, such as a smartphone or tablet terminal. The exercise motion analysis server 20 may be a server computer. Each of the user terminals 10 and the exercise motion analysis server 20 may be constituted by a computer including a CPU (Central Processing Unit), a GPU (Graphics Processing Unit), a R_AM (Random Access Memory), a ROM (Read Only Memory), an auxiliary storage device, a communication device, and the like. Each user terminal 10 and the exercise motion analysis server 20 may be constituted respectively by separate computers or may be implemented by a single computer that combines the functions of both. The present embodiment describes an example in which separate computers are provided therefor.

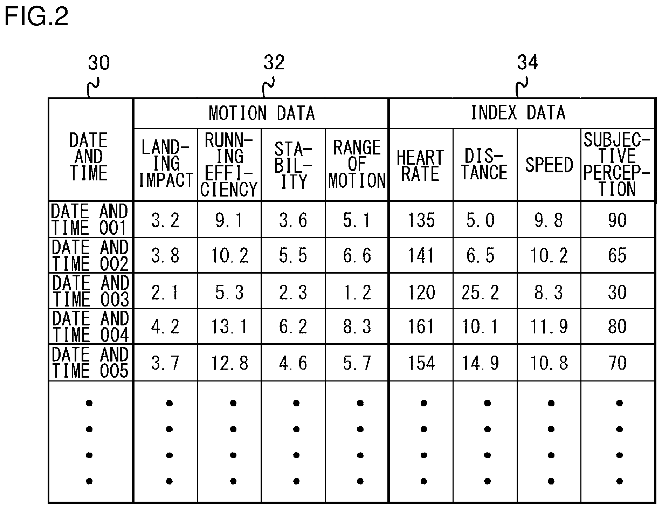

FIG. 2 shows examples of the motion data and the index data. The data shown in FIG. 2 is data that a user terminal 10 organizes or calculates based on the information acquired from the wearable device 16 and transmits to the exercise motion analysis server 20. In a modification, a user terminal 10 may transfer the information acquired from the wearable device 16 as it is to the exercise motion analysis server 20, and the exercise motion analysis server 20 may organize or calculate the data as shown in FIG. 2.

A first column 30 shows information on a date and time of acquisition of detection data. A second column 32 shows motion data. A third column 34 shows index data.

The motion data in the second column 32 include four motion parameters of "landing impact", "running efficiency", "stability", and "range of motion". The "running efficiency" may be a value indicating kicking acceleration. The "stability" may be a value indicating lateral impact. The "range of motion" may be a value indicating the amount of pelvis rotation. Further, the motion data may also include motion parameters such as "vertical motion", "resilience" indicated by vertical stiffness, and "tempo" indicated by the cadence.

The index data in the third column 34 include four index parameters of "heart rate", "reached distance", "maximum speed", and "subjective perception". Instead of the "heart rate", "oxygen uptake" calculated based on the heart rate may be included. The "subjective perception" may be a value indicating a sense of pleasure, fatigue, or pain during or after running and manually entered by a user.

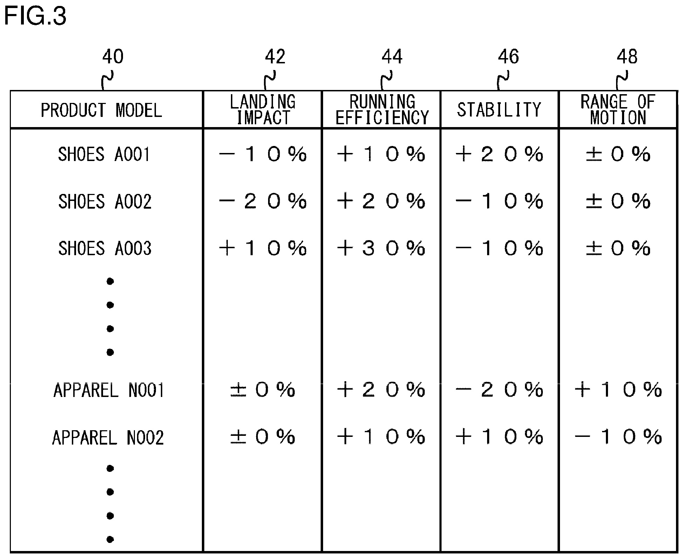

FIG. 3 shows relationships between product models of shoes and the like and motion parameter changing properties of the respective product models. The motion parameter changing properties of each product model shown in FIG. 3 are defined as wearing tool property data, which will be described later, and referred to as bases for selecting shoes and the like.

A first column 40 shows the name of a product model of shoes and the like. A second column 42 shows a change rate of the motion parameter "landing impact". A third column 44 shows a change rate of the motion parameter "running efficiency". A fourth column 46 shows a change rate of the motion parameter "stability". A fifth column 48 shows a change rate of the motion parameter "range of motion".

As the product models of shoes and the like in the first column 40, product model names of "A001", "A002", and "A003" for shoes and product model names of "N001" and "N002" for functional apparel, such as compression tights and joint supporters, are shown as examples. Each of these shoes and the like differs in terms of a motion parameter expected to change when it is worn. For example, the properties of the "Shoes A001" are shown such that, in comparison with a standard wearing tool, the "landing impact" decreases by 10%, the "running efficiency" increases by 10%, the "stability" increases by 20%, and the "range of motion" remains unchanged. The properties of the "Shoes A002" are shown such that, in comparison with a standard wearing tool, the "landing impact" decreases by 20%, the "running efficiency" increases by 20%, the "stability" decreases by 10%, and the "range of motion" remains unchanged. The properties of the "Shoes A003" are shown such that, in comparison with a standard wearing tool, the "landing impact" increases by 10%, the "running efficiency" increases by 30%, the "stability" decreases by 10%, and the "range of motion" remains unchanged.

The properties of the "Apparel N001" are shown such that, in comparison with a standard wearing tool, the "landing impact" remains unchanged, the "running efficiency" increases by 20%, the "stability" decreases by 20%, and the "range of motion" increases by 10%. The properties of the "Apparel N002" are shown such that, in comparison with a standard wearing tool, the "landing impact" remains unchanged, the "running efficiency" increases by 10%, the "stability" also increases by 10%, and the "range of motion" decreases by 10%.

Thus, a motion parameter expected to change and the change rate thereof differ for each product model of shoes and the like. Accordingly, it is conceivable, for example, that, to a user whose exercise performance is expected to be enhanced by improving the "stability" rather than improving the "landing impact", the "Shoes A001" may be recommended, and, to a user whose exercise performance is expected to be enhanced by improving the "range of motion" rather than improving the "stability", the "Apparel N001" may be recommended.

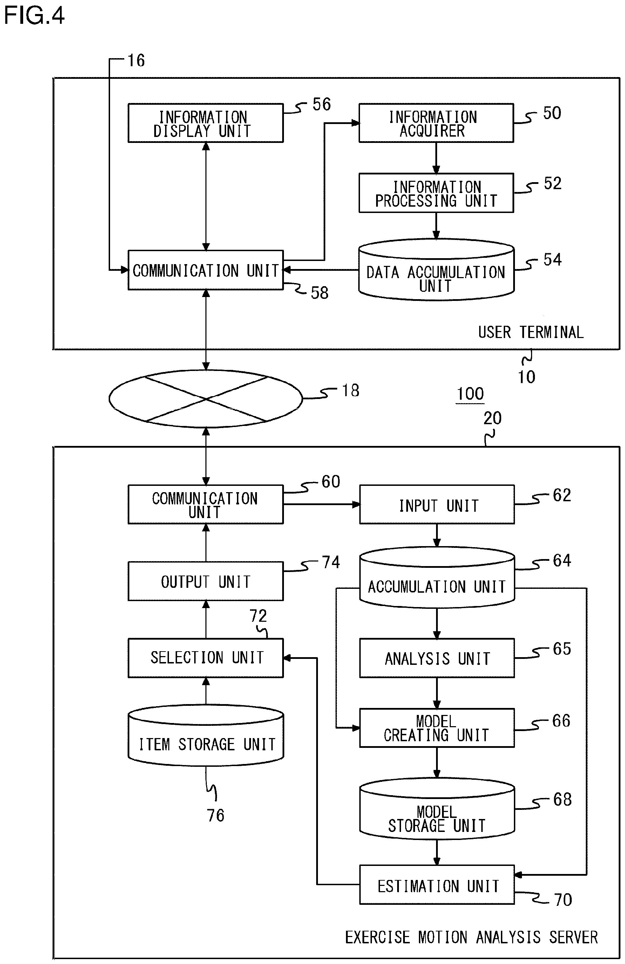

FIG. 4 is a functional block diagram that shows basic configurations of a user terminal and the exercise motion analysis server. FIG. 4 is a block diagram featuring the functions, and these functional blocks may be implemented in a variety of forms by hardware, software, or a combination thereof.

A user terminal 10 includes an information acquirer 50, an information processing unit 52, a data accumulation unit 54, an information display unit 56, and a communication unit 58. The information acquirer 50 acquires various detection data from the wearable device 16 via the communication unit 58. The information acquirer 50 also accepts operation input by a user and acquires information based on the user's subjective perception, such as the degree of fatigue and the presence or absence of body pain during or after running. Based on the detection data and subjective information acquired by the information acquirer 50, the information processing unit 52 generates the running log data, motion data, and index data and stores those data in the data accumulation unit 54. The running log data, motion data, and index data are transmitted together with user identification information to the exercise motion analysis server 20 via the communication unit 58 and the network 18.

The exercise motion analysis server 20 includes a communication unit 60, an input unit 62, an accumulation unit 64, an analysis unit 65, a model creating unit 66, a model storage unit 68, an estimation unit 70, a selection unit 72, an output unit 74, and an item storage unit 76.

The input unit 62 receives, via the communication unit 60, the running log data, motion data, index data, and user identification information transmitted from a user terminal 10. The input unit 62 accumulates the data received from the user terminal 10 in the accumulation unit 64 such that the data is related to the identification information of the user of the user terminal 10.

The analysis unit 65 performs cluster analysis on the motion data of multiple people accumulated in the accumulation unit 64 to classify the motion data into multiple runner types.

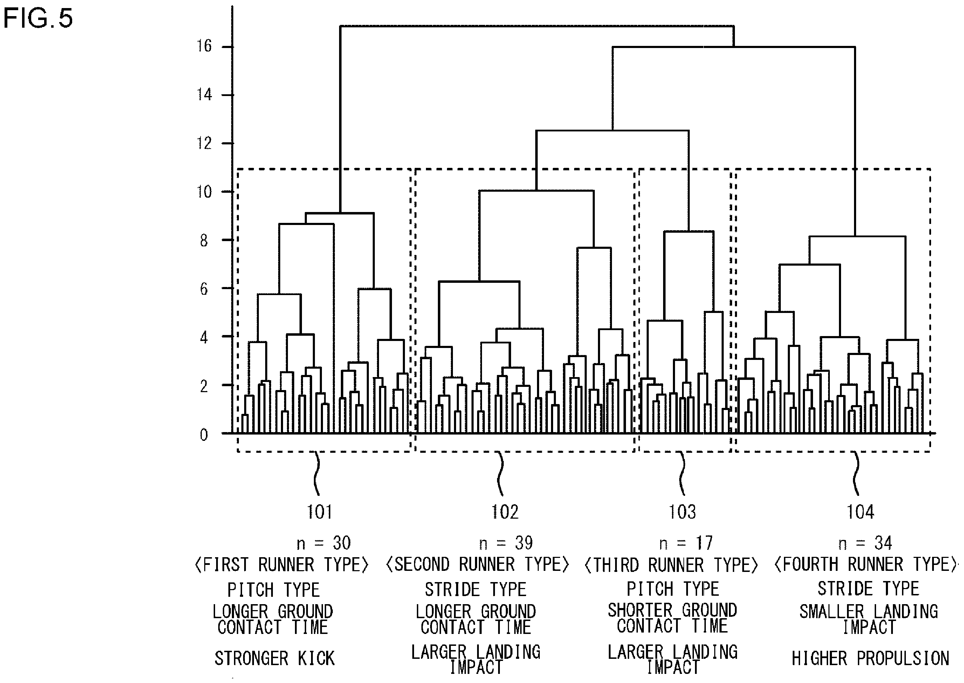

FIG. 5 is a tree diagram that shows cluster analysis of motion data using the Ward's method. FIG. 5 shows an example in which users who are similar in terms of the values of multiple types of motion parameters are connected and hierarchized and are then cut into four clusters.

A first cluster 101 is a "first runner type" formed to include 30 leaves. The motion data classified in this cluster show the motion tendencies of "pitch type", "longer ground contact time", and "stronger kick".

A second cluster 102 is a "second runner type" formed to include 39 leaves. The motion data classified in this cluster show the motion tendencies of "stride type", "longer ground contact time", and "larger landing impact".

A third cluster 103 is a "third runner type" formed to include 17 leaves. The motion data classified in this cluster show the motion tendencies of "pitch type", "shorter ground contact time", and "larger landing impact".

A fourth cluster 104 is a "fourth runner type" formed to include 34 leaves. The motion data classified in this cluster show the motion tendencies of "stride type", "smaller landing impact", and "higher propulsion".

With regard to the motion tendencies of each runner type as described above, a motion parameter in the motion data for which multiple people classified in a runner type have similar values is extracted, and a motion parameter for which particularly many people have similar values is recognized as a motion tendency of the runner type.

Referring back to FIG. 4, the model creating unit 66 creates a prediction model by machine learning using a random forest for each of multiple runner types, with the motion data of multiple people classified in the runner type as the teacher data. The prediction model thus created is stored in the model storage unit 68. By inputting the motion data of a user into such a prediction model, the estimation unit 70 estimates the runner type in which the user is classified. In a modification, machine learning with an algorithm other than random forests, such as decision trees or deep forests, may be used.

The model creating unit 66 further creates, for each runner type, a multiple regression analysis model for obtaining the degree of contribution of each motion parameter to the enhancement of exercise performance of the runner type, in which multiple types of motion parameters in the motion data of multiple people classified in the runner type are set as the explanatory variables, and an index parameter in the index data of multiple people of the same runner type is set as the objective variable. The accumulation unit 64 accumulates multiple types of index data, and each of the multiple types of index data indicates exercise performance for a different objective. The model creating unit 66 creates, individually for each of the exercise objectives, i.e., each piece of index data, a multiple regression analysis model in which the corresponding index parameter is set as the objective variable, and multiple types of motion parameters are set as the explanatory variables. The multiple regression analysis model thus created for each runner type is stored in the model storage unit 68.

The estimation unit 70 estimates, individually for each type of index parameter in the multiple regression analysis model for each runner type stored in the model storage unit 68, which of the multiple types of motion parameters has a higher degree of contribution to the enhancement of exercise performance for the user.

Among the multiple types of motion parameters, a motion parameter of which improvement contributes to the enhancement of exercise performance indicated by an index parameter is different for each user or each runner type. This is because each user or each runner type has a different physical structure (skeletal structure and muscle strength), physical ability, and running form, and each user also has a different running objective, aim, and intention, so that an index parameter of which improvement corresponds to enhancement of the performance for the user or the runner type is also different. Therefore, in the present embodiment, the motion data and the index data are accumulated for each runner type in the accumulation unit 64, and the accumulated data is subjected to multiple regression analysis using the stepwise method, so as to find out which motion parameter is likely to affect the enhancement of exercise performance for users of the runner type. Further, the type of a motion parameter that leads to the improvement of an index parameter that fits the user's intention is also found out.

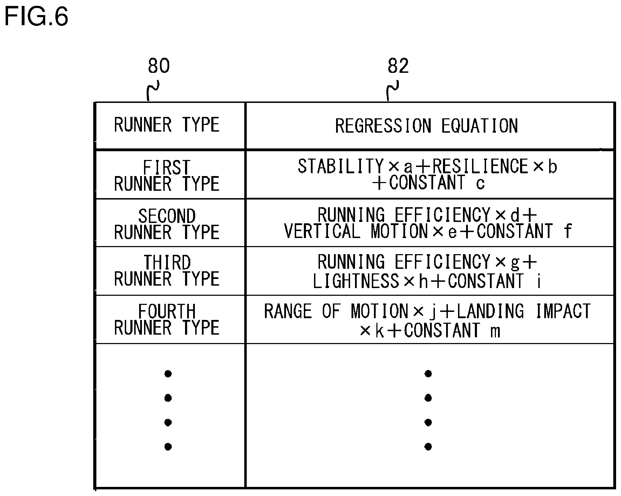

FIG. 6 shows an example of a regression equation for each runner type. A first column 80 shows a runner type. A second column 82 shows a regression equation for each runner type obtained by multiple regression analysis. In the example of FIG. 6, multiple regression analysis is performed using the stepwise method with the index parameter "oxygen uptake" as the objective variable. The more a change in a motion parameter can lower the "oxygen uptake", the more the change in the motion parameter will contribute to the enhancement of exercise performance. Experiments have shown that the explanatory rate (adjusted R²) of the index parameter estimated by using a regression equation shown in FIG. 6 was about 59-96%. Accordingly, the estimation of an index parameter based on multiple regression analysis described in the present embodiment is shown to have sufficient accuracy.

For example, from the motion data and the index data of the first runner type, the regression equation of "stability x a + resilience x b + constant c" can be obtained. As a multiple regression analysis model actually created by the model creating unit 66 for each runner type, a regression equation that includes motion parameters besides the "stability" and "resilience" is created. In such a regression equation, the estimation unit 70 narrows the multiple types of motion parameters down to a limited number of, such as one or more of, motion parameters that have particularly high ratios of contribution to the improvement of the index parameter for the runner type. FIG. 6 shows a regression equation only using the motion parameters thus narrowed down, for the sake of convenience.

The coefficient "a" of the "stability" is a negative value; accordingly, when the value of a motion parameter of the "stability", such as the "lateral impact", is larger, the index parameter "oxygen uptake" can be lowered more, which contributes to the enhancement of the exercise performance. Also, the coefficient "b" of the "resilience" is a positive value; accordingly, when the value of a motion parameter of the "resilience", such as the "vertical stiffness", is smaller, the index parameter "oxygen uptake" can be lowered more, which contributes to the enhancement of the exercise performance.

For example, from the motion data and the index data of the second runner type, the regression equation of "running efficiency x d + vertical motion x e + constant f" can be obtained. More specifically, the estimation unit 70 narrows multiple types of motion parameters down to the motion parameters of "running efficiency" and "vertical motion", as the motion parameters that have particularly high ratios of contribution to the improvement of the index parameter for the runner type; FIG. 6 shows a regression equation only using the motion parameters thus narrowed down.

The coefficient "d" of the "running efficiency" is a negative value; accordingly, when the value of a motion parameter of the "running efficiency", such as the "kicking acceleration", is larger, the index parameter "oxygen uptake" can be lowered more, which contributes to the enhancement of the exercise performance. Also, the coefficient "e" of the "vertical motion" is a positive value; accordingly, when the value of a motion parameter of the "vertical motion" is smaller, the index parameter "oxygen uptake" can be lowered more, which contributes to the enhancement of the exercise performance.

For example, from the motion data and the index data of the third runner type, the regression equation of "running efficiency x g + lightness x h + constant i" can be obtained. More specifically, the estimation unit 70 narrows multiple types of motion parameters down to the motion parameters of "running efficiency" and "lightness", as the motion parameters that have particularly high ratios of contribution to the improvement of the index parameter for the runner type; FIG. 6 shows a regression equation only using the motion parameters thus narrowed down.

The coefficient "g" of the "running efficiency" is a negative value; accordingly, when the value of a motion parameter of the "running efficiency", such as the "kicking acceleration", is larger, the index parameter "oxygen uptake" can be lowered more, which contributes to the enhancement of the exercise performance. The coefficient "h" of the "lightness" is also a negative value; accordingly, when the value of a motion parameter of the "lightness", such as the "cadence", is larger, the index parameter "oxygen uptake" can be lowered more, which contributes to the enhancement of the exercise performance.

For example, from the motion data and the index data of the fourth runner type, the regression equation of "range of motion x j + landing impact x k + constant m" can be obtained. More specifically, the estimation unit 70 narrows multiple types of motion parameters down to the motion parameters of "range of motion" and "landing impact", as the motion parameters that have particularly high ratios of contribution to the improvement of the index parameter for the runner type; FIG. 6 shows a regression equation only using the motion parameters thus narrowed down.

The coefficient "j" of the "range of motion" is a negative value; accordingly, when the value of a motion parameter of the "range of motion", such as the "amount of pelvis rotation", is larger, the index parameter "oxygen uptake" can be lowered more, which contributes to the enhancement of the exercise performance. Also, the coefficient "k" of the "landing impact" is a positive value; accordingly, when the value of a motion parameter of the "landing impact" is smaller, the index parameter "oxygen uptake" can be lowered more, which contributes to the enhancement of the exercise performance.

FIG. 6 shows an example in which the type of a motion parameter that contributes to the enhancement of exercise performance by reducing the "oxygen uptake" is estimated by multiple regression analysis with the "oxygen uptake" as the objective variable. The model creating unit 66 further performs multiple regression analysis with each of other index parameters, such as the "heart rate", "reached distance", "maximum speed", "pace reduction rate", and "subjective perception", as the objective variable and estimates the type of a motion parameter that contributes to the improvement of the index parameter. When the index parameter "heart rate" is set as the objective variable, the type of a motion parameter that contributes to reducing the "heart rate" is estimated.

When the index parameter "reached distance" is set as the objective variable, the type of a motion parameter that contributes to increasing the "reached distance" is estimated. For example, for a user who aims at the completion of a long-distance race such as a marathon, being able to run a longer distance would be beneficial and would imply the enhancement of the exercise performance. To increase the "reached distance", for example, the motion parameter "resilience" may work negatively, so that smaller "resilience" may contribute to the enhancement of the exercise performance.

When the index parameter "maximum speed" is set as the objective variable, the type of a motion parameter that contributes to increasing the "maximum speed" is estimated. For example, for a user who aims at speeding up, being able to increase the instantaneous speed would be beneficial and would imply the enhancement of the exercise performance. To increase the "maximum speed", for example, the motion parameter "resilience" may work positively, so that larger "resilience" may contribute to the enhancement of the exercise performance. In this respect, it differs from the case where the "reached distance" is set to the aim in which the same "resilience" works negatively.

When the index parameter "pace reduction rate" is set as the objective variable, the type of a motion parameter that contributes to reducing the "pace reduction rate" is estimated. For example, for a user who aims at stably running long distances, preventing the pace from slowing down would be beneficial and would imply the enhancement of the exercise performance.

When the index parameter "subjective perception" is set as the objective variable, the type of a motion parameter that contributes to reducing fatigue or body pain during or after running is estimated, or the type of a motion parameter that contributes to increasing the sense of pleasure during or after running is estimated. For example, for a user who aims at continuity of practice, being able to run without fatigue or injury or being able to run comfortably would be beneficial and would imply the enhancement of the exercise performance.

Although the present embodiment employs a method of using multiple regression analysis to estimate a motion parameter that contributes to the enhancement of exercise performance, machine learning may be used instead of multiple regression analysis. In this case, besides the motion parameters and the index parameters, attribute information, such as the gender, age, height, weight, exercise history, and race, of the user and information including the type of shoes that the user uses may also be used in combination, as the teacher data in the machine learning.

Description will be given with reference to FIG. 4 again. The item storage unit 76 stores information on multiple types of shoes and functional apparel of which wearing during running is to be recommended to a user. The item storage unit 76 also stores wearing tool property data that includes a property, determined in advance for each of multiple types of shoes and the like, indicating which of the multiple types of motion parameters is changed to contribute to the enhancement of exercise performance. The wearing tool property data include, for each of the product models of multiple types of shoes and the like, the type of a motion parameter expected to change due to the wearing of the product model and the change rate thereof, determined in advance as the motion parameter changing properties. Examples of the motion parameter changing properties shown in the wearing tool property data are shown in FIG. 3.

Based on the wearing tool property data, the selection unit 72 selects, from among multiple types of shoes and the like, shoes and the like having the property of changing a motion parameter of the type estimated by the estimation unit 70 and thereby contributing to the enhancement of exercise performance. The selection unit 72 selects shoes and the like having a higher change rate of a motion parameter of the type estimated by the estimation unit 70, based on the wearing tool property data. The selection unit 72 selects, individually for each of the user's objectives, i.e., each piece of index data, shoes and the like having the property of changing a motion parameter of the type estimated by the model creating unit 66 and thereby contributing to the enhancement of exercise performance. Multiple shoes and the like may be selected, and the priority of recommendation may be determined in descending order of the change rate. For example, with regard to all candidate shoes and the like stored in the model storage unit 68, the selection unit 72 may calculate the improvement rate of an index parameter by applying the change rate of each motion parameter to a regression equation and select candidate shoes and the like to be recommended, in descending order of the improvement rate.

For example, in the case of the first runner type, the selection unit 72 selects, from among multiple types of shoes, shoes having the property of increasing the motion parameter "stability" and the property of decreasing the motion parameter "resilience". When there are multiple candidate shoes that have both the property of increasing the "stability" and the property of decreasing the "resilience", the selection unit 72 takes into account the change rate of each candidate to select, as a first candidate, shoes and the like with the highest degree of overall contribution to the improvement of the index parameter, i.e., the highest improvement rate of the index parameter; the selection unit 72 also selects, as the second and subsequent candidates, shoes and the like with relatively low degrees of contribution or improvement rates. For each of the index parameters including the "oxygen uptake", "heart rate", "distance", "speed", and "subjective perception", the selection unit 72 selects one or more candidate shoes and the like having the motion parameter changing properties that contribute to the improvement of the index parameter for the user.

The item storage unit 76 further stores the contents of multiple types of improvement suggestions to be presented to the user. As each improvement suggestion, a sentence regarding the effect of changing the value of each motion parameter is stored in the item storage unit 76. For example, when it is estimated, with the regression equation for each runner type, that the motion parameter "landing impact" (the coefficient is a positive value) has a high degree of contribution to the improvement of an index parameter, a sentence such as "If landing impact during running is small, you can achieve speed with less energy" is stored as the content of the suggestion. Also, when it is estimated that the motion parameter "stability" (the coefficient is a negative value) has a high degree of contribution to the improvement of an index parameter, a sentence such as "If stability during running is high, you can achieve speed with less energy" is stored as the content of the suggestion. The item storage unit 76 also stores the content of an improvement suggestion regarding each motion parameter, for each of the user's objectives, i.e., for each index parameter.

The item storage unit 76 further stores the contents of multiple types of training suggestions to be presented to the user. As each training suggestion, the contents of training suggested for each motion parameter, and a sentence regarding the effect obtained when the value of the motion parameter is changed by the training are stored in the item storage unit 76. The item storage unit 76 also stores the content of a training suggestion regarding each motion parameter, for each of the user's objectives, i.e., for each index parameter.

The item storage unit 76 further stores information on multiple marathon races in which participation is to be suggested as an aim for the user, and, for each marathon race, the contents of multiple types of training suggestions to be presented to the user for preparation of the participation. The item storage unit 76 further stores, for each marathon race, a correspondence relationship indicating which of multiple types of running objectives (such as "running faster", "running longer", and "running more comfortably") the race is suitable for, information on the features of the marathon race course (such as the season of the race, slope in the course, and seasonal environmental characteristics), and information on the contents of training based on the features of the marathon race.

In a modification, the item storage unit 76 may store the type of a wearing tool having a property of changing a motion parameter such as to conform to the tendencies of multiple types of motion parameters of a runner type in the motion data classified into multiple runner types and thereby contributing to the enhancement of exercise performance, as the type of a wearing tool commensurate with the runner type. In this case, shoes and the like to be recommended can be selected simply by determining which runner type the user is classified in. More specifically, the selection unit 72 determines the runner type by inputting the user's motion data into a prediction model and selects, from among multiple types of wearing tools, a wearing tool commensurate with the runner type in which the user's motion data are classified, based on the information stored in the item storage unit 76. The item storage unit 76 may also store the type of an improvement suggestion, the type of a training suggestion, and information on a marathon race, which are commensurate with a runner type.

The output unit 74 transmits, to a user terminal 10 via the communication unit 60, the types of one or more shoes and the like selected by the selection unit 72, as the shoes and the like of which wearing is to be recommended to the user. The output unit 74 displays, for example, introductions of shoes and the like to be recommended and a suggestion for improving exercise, for each of the user's objectives. More specifically, for each index parameter serving as an index of exercise performance, the type of shoes and the like selected by the selection unit 72 and the type of a motion parameter to be desirably changed are displayed.

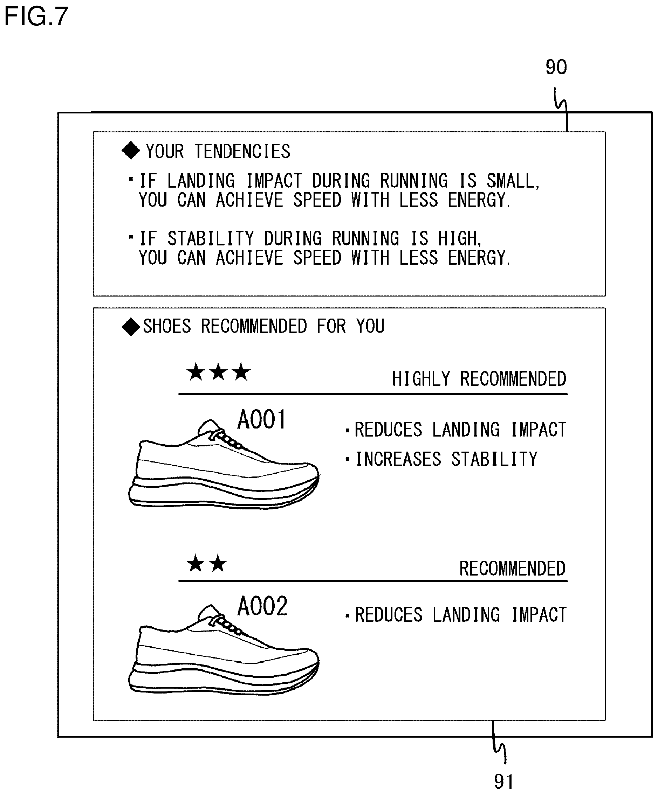

FIG. 7 shows a first screen example for recommending shoes to a user. In a first column 90, the contents of improvement suggestions are displayed. In a second column 91, the types of shoes and the like to be recommended are displayed in the order of recommendation.

In the first column 90, character strings such as "Your tendencies", "If landing impact during running is small, you can achieve speed with less energy", and "If stability during running is high, you can achieve speed with less energy" are displayed as the user's tendencies regarding motion parameters. In other words, the indication is displayed that decreasing the motion parameter "landing impact" and increasing the motion parameter "stability" increases the index parameter "maximum speed", thereby contributing to the enhancement of the exercise performance.

In the second column 91, as the contents for recommending the selected type of shoes and the like to the user, a character string such as "Shoes recommended for you" and two types of shoes to be recommended are displayed. Also, the degree of recommendation is indicated by the number of stars, and, for each of the shoes, a motion parameter expected to be improved is indicated with a character string such as "Reduces landing impact" or "Increases stability".

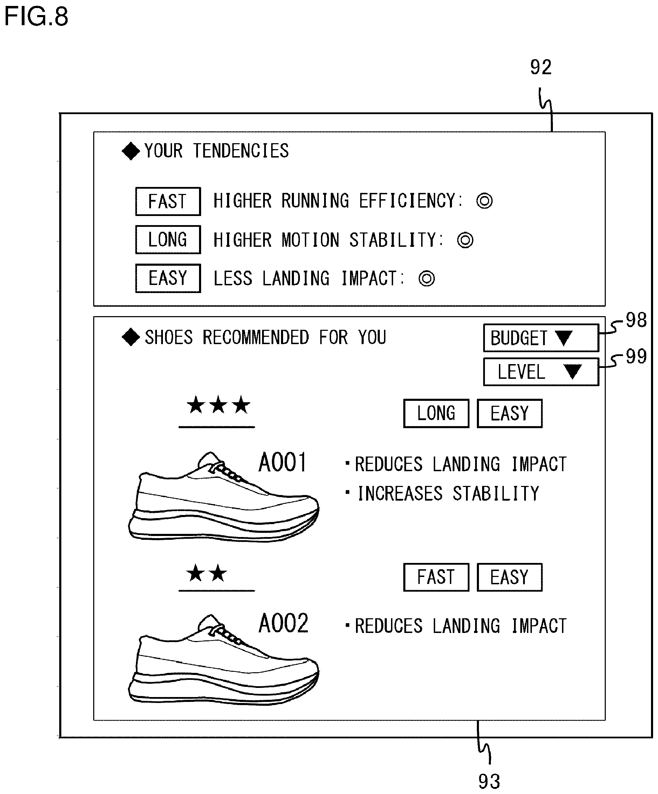

FIG. 8 shows a second screen example for recommending shoes to a user. In a first column 92, the contents of improvement suggestions are displayed. In a second column 93, the types of shoes and the like to be recommended are displayed in the order of recommendation. In the example of FIG. 8, unlike the first screen example shown in FIG. 7, a configuration is employed in which shoes and the like can be freely selected more easily based on the user's aim, budget, and running ability.

In the first column 92, as an improvement suggestion for the user, a suggestion for improving a motion parameter is displayed for each of the user's intentions. For example, a suggestion for improving a motion parameter is shown with the mark of "FAST" indicating the index parameter "maximum speed" for the aim of running faster, "LONG" indicating the index parameter "reached distance" for the aim of running longer, or "EASY" indicating the index parameter "subjective perception" for the aim of running more comfortably. For a user aiming at "FAST", indicating the "maximum speed", a character string indicating that increasing the motion parameter "running efficiency" contributes to the enhancement of the exercise performance is displayed. For a user aiming at "LONG", indicating the "reached distance", a character string indicating that increasing the motion parameter "stability" contributes to the enhancement of the exercise performance is displayed. For a user aiming at "EASY", indicating the "subjective perception", a character string indicating that decreasing the motion parameter "landing impact" contributes to the enhancement of the exercise performance is displayed.

In the second column 93, as the contents for recommending the selected type of shoes to the user, a character string such as "Shoes recommended for you" and two types of shoes to be recommended are displayed. Also, the degree of recommendation is indicated by the number of stars, and, for each of the shoes, an index parameter expected to be improved is indicated with a character string such as "FAST", "LONG", or "EASY". For example, for the first candidate shoes, three stars are shown, and, together with the words of "LONG", indicating an increase in the reached distance, and "EASY", indicating comfortable running by avoiding fatigue and pain, the motion parameters expected to be improved are indicated with character strings such as "Reduces landing impact" and "Increases stability". For the second candidate shoes, two stars are shown, and, together with the words of "FAST", indicating an increase in the maximum speed, and "EASY", indicating comfortable running by avoiding fatigue and pain, the motion parameter expected to be improved is indicated with a character string such as "Reduces landing impact".

A first menu 98 is a pull-down menu used to specify the user's budget, and a second menu 99 is a pull-down menu used to specify the user's running ability. When the user specifies his or her budget in the first menu 98 and also specifies his or her running ability in the second menu 99, the selection unit 72 narrows the candidate shoes to be displayed down to shoes that match the budget and running ability level specified by the user, so as to select and display the shoes.

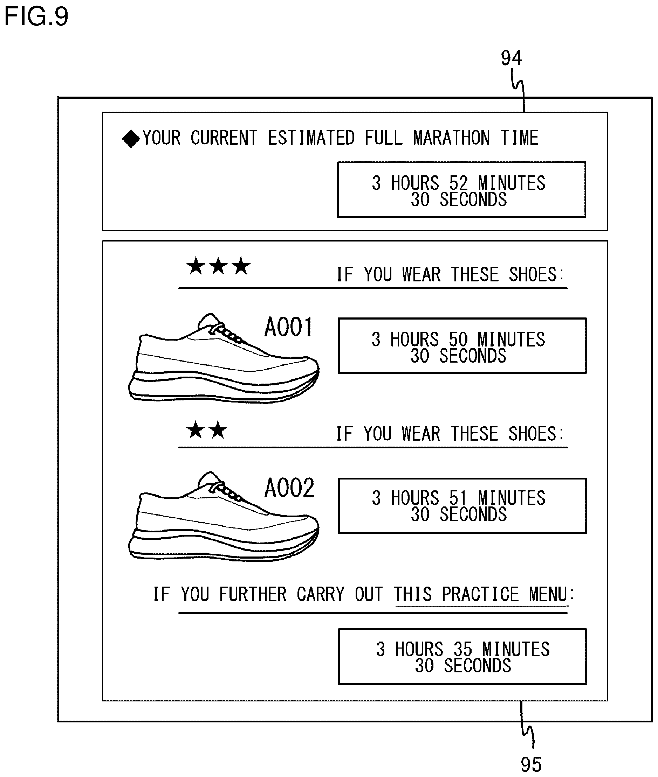

FIG. 9 shows a third screen example for recommending shoes to a user. In the example of FIG. 9, the user's current estimated marathon time is calculated and displayed, and an estimated marathon time in the case where the user wears recommended shoes is also displayed.

In a first column 94, the user's current estimated marathon time, such as "3 hours 52 minutes 30 seconds", is displayed. In a second column 95, estimated marathon times for the respective recommended shoes are displayed in the order of recommendation of the shoes. For example, with the first candidate shoes, "3 hours 50 minutes 30 seconds", which is 2 minutes shorter than the current estimated time, is displayed as an estimated marathon time in the case where the user wears the shoes. Also, with the second candidate shoes, "3 hours 51 minutes 30 seconds", which is only 1 minute shorter than the current estimated time, is displayed as an estimated marathon time in the case where the user wears the shoes. In addition, along with a text such as "If you further carry out this practice menu" linked to a practice menu page, a character string of "3 hours 35 minutes 30 seconds", which is 17 minutes shorter, is displayed as an estimated marathon time expected after the practice menu is implemented.

In this case, in order to estimate marathon times, the model creating unit 66 creates a multiple regression analysis model in which the running time is set as the objective variable, and, besides the various motion parameters in the present embodiment, parameters indicating running conditions, such as the distance, speed, pace, temperature, and altitude, are further input as the explanatory variables in multiple regression analysis. Based on such a multiple regression analysis model, the estimation unit 70 estimates a marathon time.

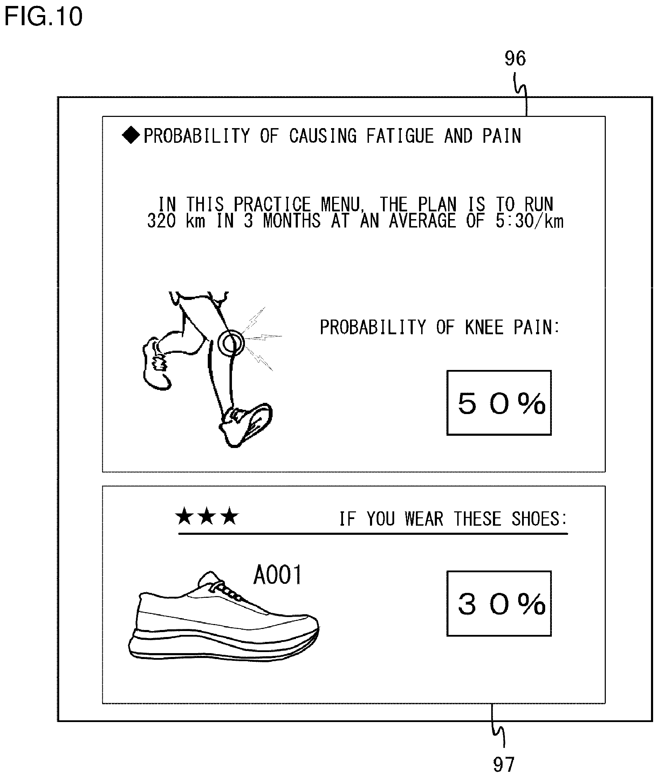

FIG. 10 shows a fourth screen example for recommending shoes to a user. In the example of FIG. 10, along with a planned running volume in a practice menu recommended in FIG. 9, the load caused by the running volume and the probability of causing pain to the body with the current running form are displayed in a first column 96. For example, with a character string such as "In this practice menu, the plan is to run 320 km in 3 months at an average of 5:30/km" and a character string such as "Probability of knee pain: 50%", the body part where the pain may occur and the probability of the pain occurrence are indicated. Also, the probability of pain occurrence in the case where the user wears recommended shoes is displayed in a second column 97. For example, with a character string such as "If you wear these shoes: 30%", the probability of pain occurrence in the case where the user wears the recommended shoes is indicated.

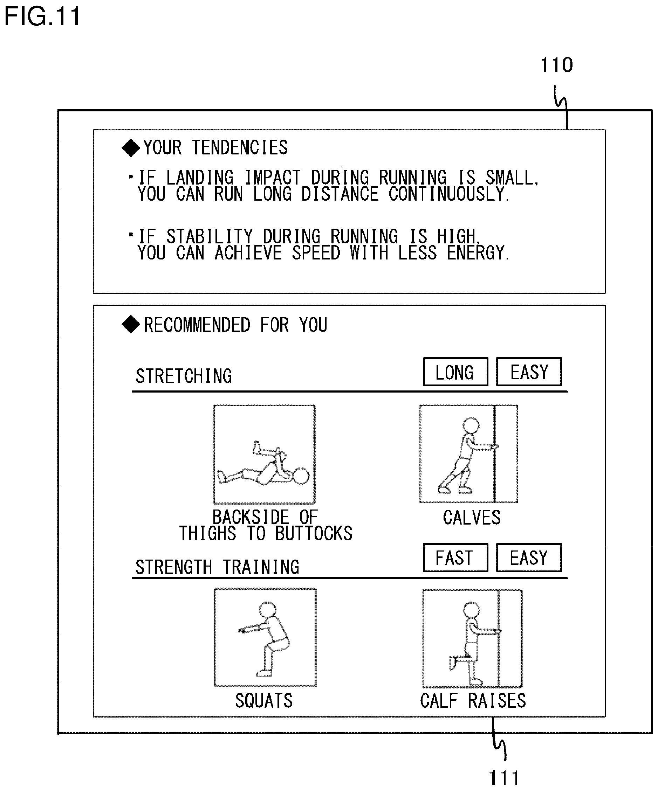

FIG. 11 shows a screen example for suggesting improvement through stretching and training to a user. In a first column 110, the contents of improvement suggestions are displayed. In a second column 111, suggestions for stretching and training are displayed.

In the first column 110, character strings such as "Your tendencies", "If landing impact during running is small, you can run long distance continuously", and "If stability during running is high, you can achieve speed with less energy" are displayed as the user's tendencies regarding motion parameters.

In the second column 111, a stretch menu is suggested with the marks of "LONG", indicating the index parameter "reached distance" for the aim of running longer, and "EASY", indicating the index parameter "subjective perception" for the aim of running more comfortably. It is indicated that, by working on this stretch menu, improvements in the index parameters "reached distance" and "subjective perception" can be expected. Also, a strength training menu is suggested with the marks of "FAST", indicating the index parameter "maximum speed" for the aim of running faster, and "EASY", indicating the index parameter "subjective perception" for the aim of running more comfortably. It is indicated that, by working on this strength training menu, improvements in the index parameters "maximum speed" and "subjective perception" can be expected.

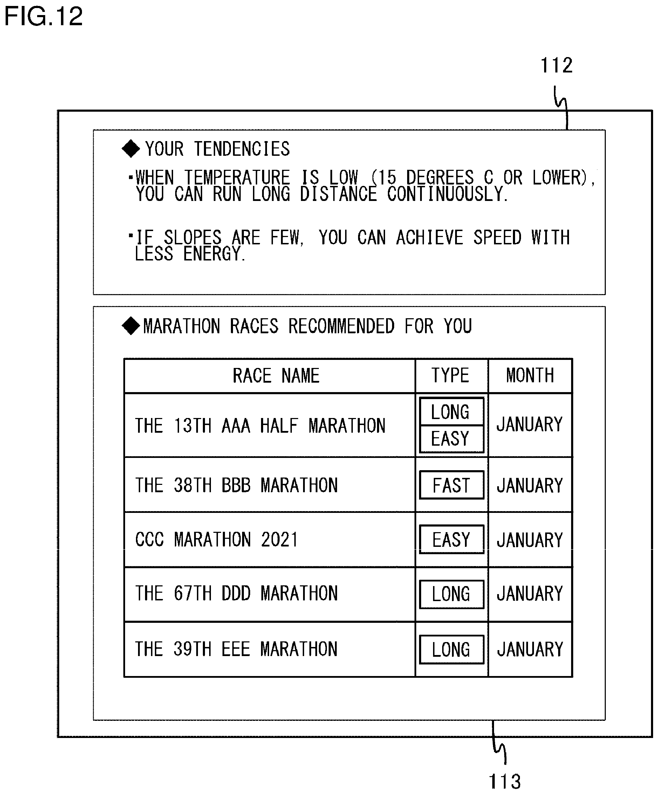

FIG. 12 shows a screen example for suggesting participation in a marathon race to a user. In a first column 112, the contents of improvement suggestions are displayed. In a second column 113, a list of suggested marathon races is displayed.

In the first column 112, character strings such as "Your tendencies", "When temperature is low (15 degrees C), you can run long distance continuously", and "If slopes are few, you can achieve speed with less energy" are displayed as the user's tendencies regarding motion parameters.

In the second column 113, a list of suggested marathon races is displayed, with each race's name, type, and month in which the race is held. In the list, as a feature of each marathon race, whether the marathon race is suitable for a user who aims at running faster, a user who aims at running longer, or a user who aims at running more comfortably is indicated by the mark "FAST", "LONG", or "EASY".

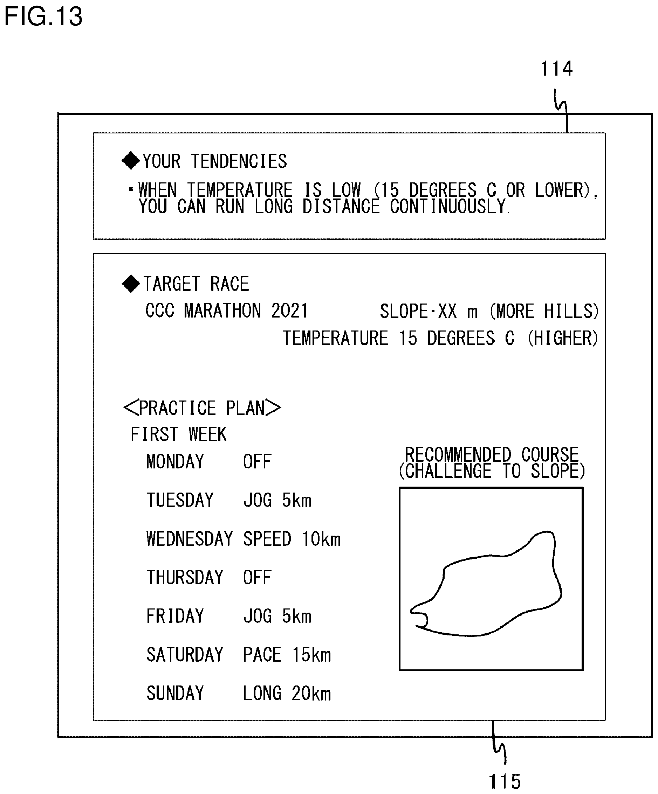

FIG. 13 shows a screen example for suggesting a practice menu to a user. In a first column 114, the contents of improvement suggestions are displayed. In a second column 115, a suggestion for a practice menu is displayed.

In the first column 114, character strings such as "Your tendencies" and "When temperature is low (15 degrees C), you can run long distance continuously" are displayed as the user's tendencies regarding motion parameters.

In the second column 115, the name of a marathon race as a target race and, as the features of the marathon race, character strings such as "Slope XX m (more hills)" and "Temperature 15 degrees C (higher)" are displayed. Also, as the "Practice plan", a practice menu and a suggested practice course are displayed.

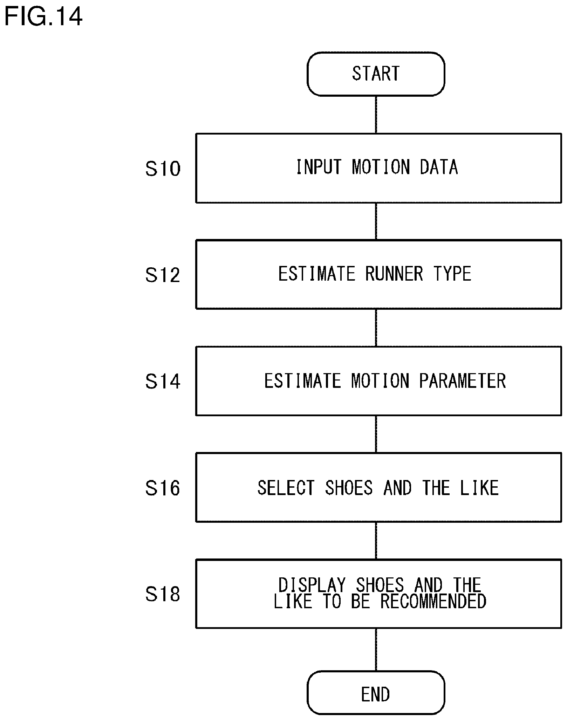

FIG. 14 is a flowchart that shows a process of selecting a wearing tool. The input unit 62 inputs the motion data and the index data of a user and accumulates the data in the accumulation unit 64 (S10). The estimation unit 70 inputs the motion data into a prediction model and estimates which of multiple runner types the user falls into (S12). Based on a multiple regression analysis model, the estimation unit 70 estimates the type of a motion parameter that contributes to the enhancement of exercise performance indicated by an index parameter for the runner type (S14). The selection unit 72 selects, from among multiple types of shoes and the like, shoes and the like that can contribute to the enhancement of exercise performance by changing a motion parameter estimated by the estimation unit 70 (S16). The output unit 74 displays, on the user terminal 10 via the network 18, the contents for recommending the selected shoes and the like to the user.

The present invention has been described with reference to an embodiment. The embodiment is intended to be illustrative only, and it will be obvious to those skilled in the art that various modifications to a combination of constituting elements or processes could be developed and that such modifications also fall within the scope of the present invention.

### INDUSTRIAL APPLICABILITY

The present invention relates to a technology for analyzing exercise motions.

### REFERENCE SIGNS LIST

- 10: user terminal
- 20: exercise motion analysis server
- 62: input unit
- 64: accumulation unit
- 65: analysis unit
- 66: model creating unit
- 70: estimation unit
- 72: selection unit
- 74: output unit
- 76: item storage unit
- 100: exercise motion analysis system

## Claims

1. An exercise motion analysis system, comprising:
an input unit that inputs motion data corresponding to a plurality of types of motion parameters indicating motion states of a user who is running;
a selection unit that selects, from among a plurality of types of wearing tools, a type of a wearing tool commensurate with a runner type in which motion data of the user is classified, among a plurality of runner types classified in advance based on cluster analysis on motion data of a plurality of people; and
an output unit that outputs the type of a wearing tool selected by the selection unit as a wearing tool of which wearing is to be recommended to the user.

2. The exercise motion analysis system according to claim 1, further comprising:
a model storage unit that stores, for each of the plurality of runner types, a prediction model created by machine learning using, as teacher data, motion data of a plurality of people classified in the runner type; and
an estimation unit that estimates the runner type by inputting motion data of the user into the prediction model.

3. The exercise motion analysis system according to claim 2, further comprising:
an analysis unit that performs cluster analysis on motion data of a plurality of people and classifies the motion data into a plurality of runner types; and
a model creating unit that creates, for each of the plurality of runner types, the prediction model by machine learning using, as teacher data, motion data of a plurality of people classified in the runner type.

4. The exercise motion analysis system according to any one of claims 1 through 3, further comprising
an item storage unit that stores a type of a wearing tool having a property of changing a motion parameter such as to conform to a tendency of a plurality of types of motion parameters of a runner type in motion data classified into the plurality of runner types and thereby contributing to enhancement of exercise performance, as a type of a wearing tool commensurate with the runner type, wherein
the selection unit selects, from among the plurality of types of wearing tools, a wearing tool commensurate with a runner type in which motion data of the user are classified, based on information stored in the item storage unit.

5. The exercise motion analysis system according to any one of claims 1 through 4, wherein
the input unit further inputs index data corresponding to an index parameter that indicates exercise performance of the user in the running, and
the selection unit selects, for each of the plurality of classified runner types, based on a multiple regression analysis model for obtaining the degree of contribution of each motion parameter to enhancement of exercise performance with the plurality of types of motion parameters in motion data of a plurality of people classified in a runner type set as the explanatory variables and with the index parameter in index data of a plurality of people classified in the runner type set as the objective variable, and based on a result of estimating which motion parameter has a higher degree of contribution to enhancement of exercise performance for a user of the runner type, a type of a wearing tool having a property of changing the estimated motion parameter and thereby contributing to enhancement of exercise performance, from among the plurality of types of wearing tools.

6. An exercise motion analysis system, comprising:
an input unit that inputs motion data corresponding to a plurality of types of motion parameters indicating motion states of a user who is running;
an analysis unit that performs cluster analysis on motion data of a plurality of people and classifies the motion data into a plurality of runner types;
a model creating unit that creates, for each of the plurality of runner types, a prediction model by machine learning using, as teacher data, motion data of a plurality of people classified in the runner type;
an estimation unit that estimates the runner type by inputting motion data of the user into the prediction model; and
an output unit that outputs information corresponding to a tendency of a value of the motion parameter in the estimated runner type.

7. An exercise motion analysis method, comprising:
inputting, by a predetermined information acquiring means, motion data corresponding to a plurality of types of motion parameters indicating motion states of a user who is running;
selecting, by predetermined processing performed by a computer and from among a plurality of types of wearing tools, a type of a wearing tool commensurate with a runner type in which motion data of the user is classified, among a plurality of runner types classified in advance based on cluster analysis on motion data of a plurality of people; and
outputting, by a predetermined information outputting means, the type of the selected wearing tool as a wearing tool of which wearing is to be recommended to the user.

8. An exercise motion analysis program causing a computer to implement:
inputting motion data corresponding to a plurality of types of motion parameters indicating motion states of a user who is running;
selecting, from among a plurality of types of wearing tools, a type of a wearing tool commensurate with a runner type in which motion data of the user is classified, among a plurality of runner types classified in advance based on cluster analysis on motion data of a plurality of people; and
outputting the type of the selected wearing tool as a wearing tool of which wearing is to be recommended to the user.
